# EUROPÄISCHE PATENTSCHRIFT

(11) **EP 1 364 045 B1**
(45) Veröffentlichungstag und Bekanntmachung des Hinweises auf die Patenterteilung: **10.09.2008**
(21) Anmeldenummer: 02700267.4
(22) Anmeldetag: 22.02.2002
(51) Int. Cl.: C12Q 1/32

(54) **Ermittlung des patientenbezogenen Kariesrisikos**
Determining the risk of caries in a patient
Détermination du risque de carie d'un patient

(30) Priorität: 23.02.2001 DE 10108900
(43) Veröffentlichungstag der Anmeldung: 26.11.2003
(73) Patentinhaber: 3M ESPE AG, 82229 Seefeld (DE)
(72) Erfinder: HÄBERLEIN, Ingo, 82363 Weilheim i. Obb. (DE); WAGNER, Ingo, 82237 Wörthsee (DE); GUGGENBERGER, Rainer, 82211 Herrsching (DE); BÖKENKAMP, Thomas, 82110 Germering (DE); STEINBEISS, Christian, 81245 München (DE); BADER-DANZIGER, Michaela, 82234 Wessling-Hochstadt (DE)
(86) Internationale Anmeldenummer: PCT/EP2002/001907
(87) Internationale Veröffentlichungsnummer: WO 2002/068679

(56) Entgegenhaltungen:
- DE-A- 19 926 728
- US-A- 4 133 875
- US-A- 4 351 899
- DATABASE CAPLUS [Online] CHEMICAL ABSTRACTS SERVICE, COLUMBUS, OHIO, US; Database accession no. 1958:106055 XP002235612 & LURA ET AL: "Acid formation in saliva and plaque, and its relation to caries activity" DEUT. ZAHN-, MUND-U. KIEFERHEILK., Bd. 28, 1958, Seiten 447-455,

## Beschreibung

Die Erfindung betrifft einen Test, der geeignet ist, das patientenbezogene Kariesrisiko durch Bestimmung von Milchsäure bzw. Lactat zu ermitteln.

### Stand der Technik

Karies ist eine Infektionskrankheit. Zu den am häufigsten genannten Leitkeimen gehören Streptococcus spp. und Lactobacillen. Kariesauslösende Mikroorganismen haben u.a. gemeinsam, dass sie Zucker, beispielsweise aus der Nahrung aufnehmen, fermentieren und als metabolische Endprodukte Säuren ausscheiden.

Prinzipiell muss zwischen der mikrobiellen Säurefreisetzung im Speichel und der Freisetzung von Säure im Zahnplaque unterschieden werden, denn die kariösen Läsionen der Zähne werden a priori von der Säure bedingt, welche durch an Zahnoberflächen haftenden Mikroorganismen, im folgenden auch als Plaque bezeichnet, gebildet werden. Die lokale Versauerung des Milieus an den Zahnoberflächen bedingt das Auflösen des Hydroxylapatits und ist damit die chemische Ursache für die Bildung von Kariesläsionen.

Wie schnell und wie stark Plaque angesäuert werden kann, hängt u.a. von der Pufferkapazität des Speichels, der Pufferkapazität der Plaqueflüssigkeit, dem mikrobiellen Bewuchs (Coogan, M.M., Motlekar, H.B. Journal of the Dental Association of South Africa 51, 823-827 (1996)) und Diffusionsprozessen (Dawes, C., Dibdin, G.H. Journal Dental Research 65, 89-94 (1986)) im Plaque ab.

Aus diesem komplexen Wechselspiel physiologischer, mikrobiologischer und chemischer Faktoren ergibt sich das Kariespotential im Plaque. Diese Faktoren liegen von Patient zu Patient unterschiedlich stark ausgeprägt vor.

Ein Ziel der modernen Zahnheilkunde ist die Einteilung der Patienten in Risikogruppen. Die Früherkennung des Kariesrisikos vor dem Auftreten von Kariesläsionen ist sinnvoll.

Die Bestimmung des patientenbezogenen Kariesrisikos mittels Plaqueanalyse hat sich in der Praxis nicht durchsetzen können, weil zur Befunderhebung eine Vielzahl von Einzelproben Plaque entnommen und analysiert werden müssten. Derartige Untersuchungen sind aufwendig, zu teuer und in der Handhabung zu komplex.

Alternativ zur Plaqueuntersuchung wird versucht, mittels Speicheluntersuchung das patientenbezogene Kariesrisiko zu ermitteln. So geht man davon aus, dass die im Speichel quantifizierbaren Mengen an Streptococcus mutans und/oder Lactobacillen mit den Mengen an Streptococcus mutans und/oder Lactobacillen in Plaque korrelieren und somit mit einer Speicheluntersuchung auf das Kariesrisiko eines Patienten geschlossen werden kann.

Zur Bestimmung von kariesauslösenden Mikroorganismen, beispielsweise Streptococcus mutans und/oder Lactobacillen in Speichel werden verschiedene Methoden angeboten.
1. Die Bestimmung von kariesauslösenden Mikroorganismen erfolgt üblicherweise nach mehrtägiger Bebrütung der Proben in geeigneten Kulturmedien, da die ursprünglich vorhandene Zahl von Mikroorganismen für eine direkte Befunderhebung nicht ausreichend ist. Nach Vermehrung der Mikroorganismen über Stunden bis Tage werden die Colony-Forming-Units (CFU) gezählt und auf die in der Probe befindlichen Zahl von Mikroorganismen geschlossen (Kneist, S.; Klein, C.; Rupf, S.; Eschrich, K. Quintessenz (1999) 50, 33-43).
   Nachteilig ist, dass die Zahl der kariösen Mikroorganismen im Speichel nur unbefriedigend mit dem Kariesrisiko korreliert, weil nicht die Anzahl der Mikroorganismen, sondern deren metabolische Aktivität entscheidend ist. Durch die Bebrütung der Proben wird femer eine Kultur pathogener Mikroorganismen angelegt, die mit den entsprechenden Vorsichtsmaßnahmen zur Risikominimierung in der Praxis behandelt werden muss. Eine besondere Entsorgung ist erforderlich. Neben diesen Nachteilen sind die Bebrütungsverfahren zur mikrobiologischen Befunderhebung teuer und sehr zeitaufwendig.
2. Immunologische Methoden sind ein weiterer Ansatz zur Bestimmung von kariesauslösenden Mikroorganismen in Speichel. Hierbei werden monoklonale oder polyklonale Antikörper gegen Oberflächenstrukturen der zu bestimmenden Mikroorganismen eingesetzt.
   Diese immunologischen Methoden sind im Vergleich zu den Bebrütungsverfahren nach Absatz 1 spezifischer, schneller und preisgünstiger, haben aber deutliche Schwächen in der Reproduzierbarkeit (Kneist, S.; Klein, C.; Rupf, S.; Eschrich, K. Quintessenz (1999) 50, 33-43). Beispielsweise befinden sich im Speichel nicht nur vitale, sondern auch erhebliche Mengen abgestorbener Mikroorganismen. Von Patient zu Patient kann das Verhältnis zwischen toten und vitalen Mikroorganismen unterschiedlich sein. Da die Antikörper nicht zwischen vitalen und toten Mikroorganismen unterscheiden können, ergibt sich eine unvorhersagbare Schwankungsbreite in der Ableitung des vorhandenen pathogenen Potentials der evaluierten Mikroorganismen (Aass, A.M.; Preus, H.R., Zambon, J.J., Gjermo, P. Scand J. Dent Res (1994) 102, 355-360). Darüber hinaus gilt auch hier die Einschränkung, dass die Zahl der kariösen Mikroorganismen im Speichel nur unbefriedigend mit dem Kariesrisiko korreliert, weil nicht die Anzahl der Mikrooganismen sondern deren metabolische Aktivität entscheidend ist.
3. Die Methode mit der höchsten Sensitivität beruht auf der Poly-Chain-Reaction-(PCR-)Technologie. Geringste Mengen Mikroorganismen können mit hoher Spezifität nachgewiesen werden. Die PCR-Technologie ist zeitaufwendig, komplex, kostenintensiv und in der Beherrschung nicht trivial (Rupf, S., Kneist, S., Merte, K., Eschrich, K. Eur. J. Oral. Sci. (1999) 107, 75-81). Auch hier muß die Einschränkung gemacht werden, dass nicht zwischen lebenden und toten Mikroorganismen unterschieden wird. Darüber hinaus gilt auch hier die zusätzliche Einschränkung, dass die Zahl der kariösen Mikroorganismen im Speichel nur unbefriedigend mit dem Kariesrisiko korreliert, weil nicht die Anzahl der Mikroorganismen, sondern deren metabolische Aktivität entscheidend ist.

Alle Methoden zur Quantifizierung von kariesauslösenden Mikroorganismen im Speichel zur Ermittlung des Kariesrisikos haben den entscheidenden Nachteil, dass die angestrebte Aussage - patientenbezogenes Kariesrisiko - nicht bzw. nur sehr unzureichend getroffen werden kann, weil aus den vorhandenen Mengen an kariesauslösenden Mikroorganismen im Speichel nicht auf deren Kariespotential in Plaque geschlossen werden kann.

Da die Speichelzusammensetzung von Patient zu Patient unterschiedlich ist und Einfluss auf die metabolische Kapazität der vorhandenen Mikroorganismen nimmt (Minah, G.E., McEnery, M.C., Flores, J.A. Archivs Oral Biol. (1986) 31, 633-638), wird empfohlen, neben der Quantifizierung von kariesauslösenden Mikroorganismen auch die durch Zucker induzierbare Versauerung des Patientenspeichels zu untersuchen. Die pH-Abnahme wird durch die mikrobielle Säurefreisetzung bedingt und wird als Maß für die metabolische Aktivität der Mikroorganismen im Speichel gesehen. Die beobachtbare Abnahme des pH-Werts im Speichel ist allerdings davon abhängig, über welche Pufferkapazität der Speichel eines Patienten verfügt. Dementsprechend wird empfohlen, die Pufferkapazität des Speichels zusätzlich mit der Quantifizierung von kariesauslösenden Mikroorganismen und pH-Messung im Speichel zur Bestimmung des patientenbezogenen Kariesrisikos zu kombinieren.

Die klinische Erfahrung zeigt jedoch, dass selbst die Kombination aus Quantifizierung von Streptococcus mutans/Lactobacillen, pH-Bestimmung und Pufferkapazität keine zuverlässige Aussage hinsichtlich des patientenbezogenen Kariesrisikos gewonnen werden kann (Kleinfelder und Kirchner "Die diagnostische Sicherheit biologischer Speicheltests zur Bestimmung des individuellen Kariesrisikos", Dtsch. Zahnärztliche Zeitschrift (1993) 48, 646-648).

In den Schriften US-A-3,332,743 und EP-A-0 097 904 wird ein weiterer Versuch beschrieben, aus der metabolischen Aktivität von Mikroorganismen im Speichel auf das patientenbezogene Kariesrisiko schließen zu können. Beobachtet wird die Kapazität des Speichels, Resazurin zu Resorufin zu reduzieren, wobei die metabolische Aktivität der Mikroorganismen mit der Bildungsrate von Resorufin korrelieren soll. Von der Bildungsrate von Resorufin wird auf das patientenbezogene Kariesrisiko geschlossen. Die Reduktion von Resazurin zu Resorufin erfolgt durch mikrobielle Dehydrogenasen. Nicht nur Streptococcus mutans oder Lactobacillen verfügen über Dehydrogenasen, die Resazurin zu Resorufin reduzieren können. Deshalb wird der Resazurin/Resorufin-Test allgemein als Vitalitätstest für Mikroorganismen eingesetzt und ist demnach nicht spezifisch für die pathogenen Karieserreger, die via Säurefreisetzung zur Versauerung des Mundmilieus beitragen, und ist damit für die Ermittlung des patientenbezogenen Kariesrisikos unbrauchbar.

Eine weitere in der Literatur diskutierte Möglichkeit, die zahnhartsubstanzschädigende metabolische Aktivität kariesauslösender Mikroorganismen zu ermitteln, ist die Freisetzung von Säuren, beispielsweise Ameisensäure, Essigsäure, Propionsäure, Milchsäure. Welche Säuren maßgeblich an der Schädigung der Zahnhartsubstanz beteiligt sind, hängt beispielsweise von der Nährstoffversorgung ab. Wenn ausreichend Glukose vorhanden ist, sezemieren Streptococci bevorzugt Milchsäure, wohingegen bei limitiertem Glukoseangebot bevorzugt Essigsäure, Propionsäure und Ameisensäure gebildet werden.

In den Patentschriften US-A-4,351,899 und US-A-4,254,222 wird eine Methode zur enzymatischen Bestimmung von Milchsäure in biologischen Flüssigkeiten, vorzugsweise Blut, beschrieben. Durch enzymatische Reduktion von Milchsäure durch eine Dehydrogenase wird neben Pyruvat Nicotinamid-Adenin-Dinucleotid (NADH) gebildet. NADH wird anschließend mit Redoxindikatoren, vorzugsweise Tetrazoliumderivate und Phenazinderviaten, bestimmt. Die Übertragung dieses Prozesses auf Speichel würde eine schnelle und einfache Methode zur Bestimmung der metabolischen Aktivität von kariogenen Mikroorganismen und damit des Kariesrisikos ermöglichen. Der in diesen Schriften beschriebene Prozess ist jedoch in Speichel nicht anwendbar, da eine im Speichel vorkommende Nebenreaktion die Signalgebung durch die Redoxindikatoren maßgeblich verfälscht. Ohne Zugabe der für die Nachweisreaktion benötigten Dehydrogenase werden durch Speichelkomponenten in Kombination mit NAD die Redoxindikatoren bereits reduziert und Signale erzeugt, die nicht auf die Gegenwart von Lactat im Speichel zurückzuführen sind.

### Aufgabe

Aufgabe der vorliegenden Erfindung ist es daher, ein Verfahren bereitzustellen, mit dem die Einschätzung des vorherrschenden patientenbezogenen Kariesrisikos ermöglicht wird und dabei die bekannten Nachteile des Standes der Technik vermieden werden.

### Lösung

Überraschenderweise wurde gefunden, dass mit dem im Rahmen des erfindungsgemäßen Verfahrens verwendeten Test die Patienten mit erhöhtem Kariesrisiko identifiziert werden können.

Erfindungsgemäß ist auch die Kombination dieses Tests mit diagnostischen Abformungen, beispielsweise solchen aus der DE-A-199 26 728. Hiermit gelingt die Unterscheidung zwischen solchen Patienten, die zwar im Speichel oder auf der Zunge ein Kariesrisiko aufweisen, aber dennoch auf den Zähnen kein Plaque mit Kariesrisiko haben, und denen, die ein tatsächliches Kariesrisiko aufweisen.

Der Vorteil der Erfindung liegt außerdem darin, dass eine eindeutige Ermittlung des Kariesrisikos eines Patienten erfolgen, eine zielgerichtete Medikamentierung abgeleitet und eine Über- bzw. Unterbehandlung vermieden werden kann.

Ferner liegt der Vorteil der Erfindung darin, dass mit dem Speicheltest der Bedarf weitergehender Untersuchungen, beispielsweise einer diagnostischen Abformung, festgestellt werden kann, und zwar zu Beginn der Behandlung, beispielsweise im Rahmen der Gruppenprophylaxe in Schulen und Kindergärten. Vorteilhaft ist ferner die einfache Kontrolle des Behandlungsverlaufs.

Unter dem Begriff Kariesrisiko sind im Rahmen dieser Erfindung sowohl das Grundrisiko des Patienten, an Karies zu erkranken, als auch das Tageszeit- und Emährungsgewohnheiten-abhängige Risiko zu verstehen. Vorteilhafterweise führt der Patient zur Bestimmung des Grundrisikos, das durch die grundsätzliche Zusammensetzung der Mundflora festgelegt ist, einfache Reinigungsmaßnahmen, wie beispielsweise Spülen und/oder Zähneputzen, bevorzugt Zähneputzen, vor Anwendung des erfindungsgemäßen Verfahrens durch. Die Reinigungsmaßnahme ist bei der Bestimmung des Tageszeit- und Ernährungsgewohnheiten-abhängigen Risikos nicht unbedingt durchzuführen, sollte in einer bevorzugten Ausführungsform des erfindungsgemäßen Verfahrens aber dennoch erfolgen.

Die Begriffe "umfassend" und "enthaltend" leiten im Rahmen dieser Schrift eine nicht-abschließende Aufzählung ein.

Der erfindungsgemäß verwendete Speicheltest beruht auf dem überraschenden Effekt, dass sich unter Einhaltung entsprechender Vorkehrungen die Milchsäurebildung als Maß für die metabolische Aktivität von Mikroorganismen im Speichel hervorragend zur Bestimmung des vorherrschenden patientenbezogenen Kariesrisikos eignet und durch Quantifizierung bzw. Semi-Quantifizierung der Milchsäure ermittelt werden kann.

Im Rahmen der vorliegenden Erfindung wird unter dem Begriff "Lactat" L(+)-Lactat, unter dem Begriff Lactatdehydrogenase (LDH) die L(+)-Lactatdehydrogenase verstanden.

Zum Nachweis der Milchsäure kann diese prinzipiell in chemische, biochemische oder physikalische Reaktionen eingebunden werden, wobei wahrnehmbare Signale generiert werden.

Es wurde überraschend festgestellt, dass die unerwünschte Nebenreaktion zwischen Redoxindikatoren und Speichelkomponenten durch Einhaltung von bestimmten Mengenverhältnissen zwischen Lactatdehydrogenase, Speichel und einem Inhibitor, nämlich Pyruvat, unterdrückbar ist. Dieser Befund ist insofern überraschend, weil Pyruvat ein für die Lactatdehydrogenase bekannter Inhibitor ist (siehe z.B. Williams, R. A., Andrews, P., Biochem. J. 236, 721-727 (1986); Oba, K., Mura Kami, S., Uritani, I., J. Biochem. 81, 1193-1201 (1977)) . Für den Fachmann ist es eine nicht vorhersehbare Lösung, dass entgegen der technischen Lehre das Messenzym, hier Lactatdehydrogenase, in Gegenwart eines bekannten Inhibitors eingesetzt werden muss, um die unspezifische Nebenreaktion des Speichels soweit unterdrücken zu können, dass eine enzymatische Bestimmung der Milchsäure mittels Lactatdehydrogenase und Redoxindikatoren gelingt. Unter dem Begriff Pyruvat ist hier die Brenztraubensäure oder ihre Salze, insbesondere ihr Na- oder K-Salz, zu verstehen.

Zur enzymatischen Bestimmung oder Milchsäure in Speichel mittels Lactatdehydrogenase, die beispielsweise aus Bakterien, bevorzugt aus Lactobacillen, Streptococcen oder Staphylococcen, Tieren, bevorzugt aus Schwein, Rind, Huhn, Kaninchen, Pflanzen oder menschlichem Gewebe, beispielsweise Plazenta gewonnen werden kann, sind geeignete Mengenverhältnisse zwischen Speichel und Lactatdehydrogenase einzuhalten. Pro 0,1 ml Speichel sollten zwischen 0,001 und 100 Units Lactatdehydrogenase, bevorzugt zwischen 0,01 und 10 Units Lactatdehydrogenase und besonders bevorzugt zwischen 0,05 und 1 Units Lactatdehydrogenase eingesetzt werden.

Zusätzlich ist Pyruvat in Konzentrationen zwischen 0,001 µmol und 5 µmol pro 0,1 ml Speichel, bevorzugt zwischen 0,01 µmol und 2,5 µmol pro 0,1 ml Speichel und besonders bevorzugt zwischen 0,01 µmol und 1 µmol pro 0,1 ml Speichel einzusetzen.

Unter 1 Unit Lactatdehydrogenase ist der Umsatz von 1 µmol Pyruvat pro Minute und mg Protein zu verstehen.

Erfindungsgemäß sind beispielsweise Redoxindikatoren aus der folgenden Gruppe verwendbar: Methylenblau, 5-Cyano-2,3-ditolyl-tetrazolium-chlorid (CTC), 2-(4-lodophenyl)-3-(4-nitrophenyl)-5-phenyl-2H-tetrazolium-chlorid (INT), 8-Dimethylamino-2,3-benzophenoxazin (Meldola's blue), 1-Methoxy-phenazin-methosulphat (MPMS), 5-(3-Carboxymethoxyphenyl)-2-(4,5-dimethylthiazolyl)-3-(4-sulphophenyl)-tetrazolium (MTS), 3-(4,5-Dimethylthiazol-2-yl)-2,5 diphenyltetrazolium-bromid (MTT), 3,3'-(3,3'-Dimethoxy-4,4'-biphenylen)-bis[2-(4-nitrophenyl-5-phenyl)]-2H-tetrazolium-chlorid (NBT), Nitro-tetrazolium-violett (NTV), Phenazin-methosulphat (PMS), Natrium-3'-[1-[(phenylamino)carbonyl]-3,4-tetrazolium]bis(4-methoxy-6-nitro)-benzolsulfonsäure (XTT), Phenazin-ethosulfat (PES, WST-1).

Bevorzugt sind hierbei Meldola's blue, PES, PMS, MTS, MTT, XTT; besonders bevorzugt PES, PMS, MTS und MTT.

Die Konzentrationen der Redoxindikatoren in der Testmischung liegen zwischen 0,001 und 10 mmol/l, bevorzugt zwischen 0,01 und 5 mmol/l und besonders bevorzugt zwischen 0,05 und 2 mmol/l.

Je nach Bedarf können die Redoxindikatoren in fester Form, beispielsweise als Pulver, Granulat oder Tablette zum Test gegeben werden.

Vorzugsweise wird gemäß der Erfindung NAD in nicht limitierenden, aber auch nicht inhibierenden Mengen, beispielsweise in Konzentrationen zwischen 0,01 und 10 mmol/l, bevorzugt zwischen 0,05 und 5 mmol/l und besonders bevorzugt zwischen 0,1 und 1 mmol/l, bezogen auf die gesamte Testmischung, eingesetzt.

Je nach Bedarf kann NAD in fester Form, beispielsweise als Pulver, Granulat oder Tablette zum Test gegeben werden.

Lactatdehydrogenasen aus Bakterien, Tieren und Pflanzen verfügen über pH-Optima zwischen 4,0 und 10,0. Je nach eingesetzter Lactatdehydrogenase müssen geeignete Pufferlösungen mit unterschiedlichen pH-Werten eingesetzt werden. Als Puffersubstanzen eignen sich insbesondere Vertreter der folgenden Gruppen:
- Phosphate, beispielsweise Natriumphosphate, Natriumhydrogenphosphat, Natriumdihydrogenphophat, Kaliumphosphat, Kaliumhydrogenphosphat, Kaliumdihydrogenphosphat, Pyrophosphat;
- Carbonate, beispielsweise Natriumcarbonat, Kaliumcarbonat, Natriumhydrogencarbonat, Kaliumhydrogencarbonat;
- Essigsäure/Acetat, Citronensäure/Citrat, Diethylbarbitursäure, Tris(hydroxymethyl)aminomethan (TRIS), Glycin, Glycylglycin (Glygly), N-(2-Acetamido)-2-aminoethansulfonsäure (ACES), N-(2-Acetamido)imino-diacetat(ADA), N,N-Bis(2-hydroxyethyl)-2-aminoethansulfonsäure (BES), N,N-Bis(2-hydroxyethyl)glycin (BICINE), 2,2-Bis-(hydroxyethyl)-iminotris-(hydroxymethyl)methan (BIS-TRIS), 2-(Cyclohexylamino)ethansulfonsäure (CHES), 2-[4-(2-Hydroxyethyl-1-piperazin)]ethansulfonsäure (HEPES), 3-[4-(2-Hydroxyethyl-1-piperazinyl)]propansulfonsäure (HEPPS), 2-Morpholinoethansulfonsäure (MES), 3-Morpholinopropansulfonsäure (MOPS), Piperazin-1,4-bis(2-ethansulfonsäure) (PIPES), N-[Tris(hydroxymethyl)-methyl]-2-aminoethansulfonsäure (TES), N-[Tris(hydroxymethyl)-methyl]-glycin (TRICINE).

Besonders bevorzugt sind Phosphatpuffer, Tris-Puffer, Glygly-Puffer und Essigsäure/Acetat-Puffer.

Je nach Nachweisreaktion kann ein pH-Wert zwischen 1 und 12 erforderlich sein. Die Konzentrationen der Pufferlösungen können je nach Bedarf zwischen 0,001 mol/l und 5,0 mol/l liegen, bevorzugt zwischen 0,01 mol/l und 1,0 mol/l, besonders bevorzugt zwischen 0,1 mol/l und 0,5 mol/l.

Die pH-Einstellung der Reaktionslösung kann auch dadurch erreicht werden, dass je nach Bedarf definierte Mengen Puffersubstanzen in fester Form, beispielsweise als Pulver oder Tablette zum Test gegeben werden. Sollte es erforderlich sein, können Puffersubstanzen auch in flüssiger Form zum Test gegeben werden.

Puffersubstanzen, Redoxindikatoren, NAD und Lactatdehydrogenase sowie andere Produktionshilfsstoffe können als Kombinationen in fester Form oder als Kombination in fester und flüssiger Form zugesetzt werden, beispielsweise als Pulver in einem Bereich zwischen 1 mg und 20 g, bevorzugt zwischen 10 mg und 10 g, weiter bevorzugt zwischen 50 mg und 5 g und besonders bevorzugt zwischen 100 mg und 1 g oder in Tablettenform, beispielsweise zwischen 1 bis 20 Tabletten, bevorzugt zwischen 2 und 10 Tabletten, besonders bevorzugt zwischen 3 und 5 Tabletten. Das Gewicht der Tabletten kann beispielsweise zwischen 1 mg und 2 g, bevorzugt zwischen 10 mg und 1,5 g und besonders bevorzugt zwischen 100 mg und 1 g liegen.

Unter Produktionshilfsstoffen sind beispielsweise Hilfsstoffe, die aus der Galenik bekannt sind, zu verstehen, beispielsweise Dextrane, Zucker, Aluminiumoxid, Gläser, Quarze, Cellulose-Derivate, Wachse, Fette, Salze.

Puffersubstanzen, Redoxindikatoren, NAD und Lactatdehydrogenase können auch in flüssiger Form zur Speichelprobe zugesetzt werden, wobei sie dann entweder direkt eingesetzt werden können, wenn diese Flüssigkeiten sind, oder zuvor in Lösungsmittel, wie Wasser, Ethanol, Propanol, gelöst werden. Eingesetzt werden können Volumina zwischen 0,001 ml und 20 ml, bevorzugt zwischen 0,01 und 10 ml und besonders bevorzugt zwischen 0,1 ml und 1 ml.

Die Bildung der Milchsäure und des wahmehmbaren Signals kann in einem kontinuierlichen, parallel ablaufenden Prozess wahrgenommen werden, wobei beispielsweise nach einem definierten Zeitintervall zwischen 0,01 min und 60 min, bevorzugt zwischen 0,1 min und 30 min, besonders bevorzugt zwischen 1 min und 10 min das wahrnehmbare Signal evaluiert oder die Bildung des nachzuweisenden Stoffwechselproduktes und/oder des wahrnehmbaren Signals terminiert wird.

Zur Terminierung kommen beispielsweise Stoppreagenzien, wie Inhibitoren, beispielsweise Phosphat oder eine plötzliche pH-Änderung, beispielsweise initiiert durch Salzsäure oder Natronlauge, oder die Zugabe von Proteasen oder Tensiden in Frage.

Die Bildung der Milchsäure kann auch in einem eigenständigen Inkubationsschritt erfolgen, wobei beispielsweise nach einem definierten Zeitintervall zwischen 0,01 min und 60 min, bevorzugt zwischen 0,1 min und 30 min, besonders bevorzugt zwischen 1 min und 10 min die Bildung der Milchsäure terminiert wird.

Zur Terminierung kommen beispielsweise Stoppreagenzien, wie oben beschrieben, oder Erhitzen der Probe auf Temperaturen zwischen 50 und 100 °C oder eine Kombination aus Stoppreagenz und Temperaturerhöhung in Frage.

Zum Terminieren der Bildung von Milchsäure durch die Mikroorganismen können unterschiedlichste Methoden zur Anwendung kommen, beispielsweise
- Bestrahlen mit energiereichem Licht, wie UV
- radioaktive Behandlung
- Hitzebehandlung
- pH-Änderung, beispielsweise durch Zugabe von Säuren oder Basen,
- Zugabe von Tensiden,
- Zugabe von Inhibitoren, wie Schwermetallsalzen, Alkylierungsmittel,
- Zugabe von Mikroorganismen zerstörende Substanzen, wie Lysozym, Triclosan, Chlorhexidin, Taurolidin,
- Entfernen der Mikroorganismen aus der zu untersuchenden Speichelprobe, beispielsweise durch Ultrafiltration, Mikrofiltration mit Sterilfiltern oder Zentrifugation
oder eine geeignete Kombination aus zwei oder mehr dieser Methoden.

Zur Durchführung des erfindungsgemäßen Tests werden beispielsweise zwischen 0,001 ml und 20 ml, bevorzugt zwischen 0,01 ml und 10 ml, besonders bevorzugt zwischen 0,1 ml und 1 ml Speichel in ein Reaktionsgefäß überführt. Es muss allgemein mindestens soviel Speichel eingesetzt werden, dass durch signalerzeugende Zusätze ein wahrnehmbares Signal zur Lactat-Bestimmung erhalten wird.

Es kann auch vorteilhaft sein, Mikroorganismen aus Teilen des Mundraumes durch mechanische Einwirkung in den Speichel zu überführen und im Anschluss die Speichelprobe in ein Reaktionsgefäß zu überführen. Beispielsweise kann mit einem geeigneten Gegenstand, wie einem Stäbchen oder Schaber, Belag von der Zunge gelöst und dann erst die Probe genommen werden.

Es ist auch möglich, anstelle einer direkten Speichelprobe eine Probe von Mikroorganismen aus einer geeigneten Stelle im Mundraum mittels geeigneter Entnahmevorrichtungen zu entnehmen und diese anstelle des Speichels dem zugrunde liegenden erfindungsgemäßen Verfahren zuzuführen. Bevorzugt ist hierbei die Entnahme des Zungenbelags von 0,01 und 25 cm², bevorzugt 0,1 und 10 cm² und besonders bevorzugt zwischen 1 und 5 cm² Zungenoberfläche mittels Wattestäbchen, Vliesen oder anderen saugfähigen Materialien und Vorrichtungen. Es muss allgemein der Zungenbelag von mindestens soviel Zungenoberfläche aufgenommen werden, dass durch signalerzeugende Zusätze ein wahrnehmbares Signal zur Lactat-Bestimmung erhalten wird.

Es kann vorteilhaft sein, dass zur Normierung des wahrnehmbaren Signals im patientenbezogenen Patientenspeichel Substanzen zum Speichel gegeben werden, die die maximal mögliche Milchsäurebildung ermöglichen, beispielsweise Zucker, bevorzugt Saccharose, Glukose. Diese Substanzen können entweder in fester oder flüssiger oder einer Kombination aus fester und flüssiger Form eingebracht werden.

Möglich ist beispielsweise eine Mundspülung mit einer Zuckerlösung, wie wässrigen Lösungen von beispielsweise Glukose, Fruktose, Galaktose, Saccharose, Maltose mit einem Gehalt zwischen 0,1 und 70 % Zucker, bevorzugt zwischen 1 und 50 % Zucker und besonders bevorzugt zwischen 5 und 30 % Zucker vor der Speichelprobennahme oder der Zungenbelagentnahme.

Es kann ebenfalls vorteilhaft sein, zur Speichelprobe selbst Zucker, wie Glukose, Fruktose, Galaktose, Saccharose, Maltose, bis zur einer Sättigung zwischen 0,1 und 70 %, bevorzugt 1 und 60 % und besonders bevorzugt 10 und 50 % zu geben.

Es kann auch vorteilhaft sein, Zucker zwischen 0,1 und 10 g, bevorzugt zwischen 0,5 und 5 g und besonders bevorzugt zwischen 1 und 2 g im Mundraum in Form von beispielsweise Zuckerwürfel, Zuckerpulver oder Zuckertablette vor der Speichelprobennahme und der Zungenbelagentnahme zergehen zu lassen.

Es kann vorteilhaft sein, zur Zungenbelagentnahme mittels Wattestäbchen, Vliesen oder anderen saugfähigen Materialien diese mit einer Lösung, enthaltend zwischen 0,1 und 70 % Zucker, bevorzugt zwischen 1 und 50 % Zucker und besonders bevorzugt zwischen 5 und 30 % Zucker zu tränken und nach dem Trocknen zu verwenden.

In einer ebenfalls bevorzugten Ausführungsform des erfindungsgemäßen Verfahrens können die beispielsweise zur Zungenbelagentnahme verwendeten Wattestäbchen, Vliese oder anderen saugfähigen Materialien einen oder mehrere diagnostische Bestandteile wie beispielsweise Saccharose, NAD, MTP, PMS, LDH oder Pyruvat enthalten, wie sie obenstehend beschrieben sind. Unter anderem bevorzugt enthalten ist NAD.

In einer besonders bevorzugten Ausführungsform enthalten die beispielsweise zur Zungenbelagentnahme verwendeten Wattestäbchen, Vliese oder anderen saugfähigen Materialien sowohl mindestens eine Zuckerlösung, wie oben beschrieben, und mindestens einen diagnostischen Bestandteil wie beispielsweise NAD.

Die Erfindung kann in der Praxis beispielsweise im Rahmen einer Blisterverpackung, wie sie beispielsweise in DE 297 142 46 U beschrieben ist, angeboten werden. Diese Darreichungsart ermöglicht das Durchführen der erfindungsgemäßen Verfahren in besonders einfacher Weise.

Ebenfalls Gegenstand der vorliegenden Erfindung ist ein Verfahren zur ortsspezifischen Bestimmung des patientenbezogenen Kariesrisikos unter Verwendung einer diagnostischen Abformmasse.

Hierbei wird zunächst das patientenbezogene Kariesrisiko mittels dem vorher beschriebenen erfindungsgemäßen Verfahren bestimmt und bei positivem Risiko ein Abdruck mindestens einer Kieferregion, beispielsweise des Unter- und/oder Oberkiefers, mit einer diagnostischen Abformmasse genommen.

Als diagnostische Abformmassen sind beispielsweise solche aus der DE-A-199 26 728, insbesondere auf Alginat-, Polyether-, Silikon- oder Polyethersilikonbasis geeignet. Es handelt sich hierbei um härtbare oder filmbildende Trägermaterialien, die diagnostisch nutzbare Zusatzstoffe für die orts- und stoffspezifische intraorale Diagnose in einer bevorzugten Menge von 0,0001 bis 10 Gew.-% enthalten. Als diagnostische Zusätze sind gemäß dieser Schrift Farbstoffindikatoren, Antikörper, Enzyme und alle anderen Substanzen geeignet, die dem mit der Entwicklung von diagnostischen Testsystemen vertrauten Fachmann geläufig sind.

Derartige Abformmassen zeigen im ausgehärteten Abdruckmaterial an denjenigen Stellen ein wahrnehmbares Signal - beispielsweise eine Färbung - an denen ein erhöhtes Kariesrisiko vorliegt. Hierzu werden selektiv Stoffwechselprodukte der kariesfördernden Mikroorganismen als aktive Spezies im angewandten Nachweisverfahren benutzt.

Eingesetzt werden kann hierbei beispielsweise eine vorformulierte diagnostische Abformmasse. Ebenso kann aber auch eine herkömmliche Abformmasse verwendet werden, die durch Zugabe von diagnostisch wirksamen Substanzen durch den Behandler in eine Abformmasse mit diagnostischer Funktion überführt werden kann.

Die Erfindung wird nachfolgend durch Beispiele näher erläutert, ohne dass sie durch diese beschränkt werden soll.

### Beispiele

### Anwendungsbeispiel 1

Zum Anmischen der signalerzeugenden Lösung werden in ein Kunststoffröhrchen (2,5 ml) mit Schraubverschluss mit einer Pipette 0,1 ml NAD-Lösung (Stammlösung 30 mmol/l NAD in Glygly-Puffer 50 mmol/l, pH 9,0), 0,1 ml MTT (Stammlösung 1,5 mmol/l MTT in Glygly-Puffer 50 mmol/l, pH 9,0), 0,1 ml PMS-Lösung (Stammlösung PMS 1,0 mmol/l in Glygly-Puffer 50 mmol/l, pH 9,0), 0,1 ml LDH-Lösung (Stammlösung Lactatdehydrogenase (LDH) 60 Units/ml in Glygly-Puffer 50 mmol/l, pH 9,0), 0,1 ml Pyruvat-Lösung (Stammlösung Pyruvat 25 mmol/l Pyruvat in Glygly-Puffer 50 mmol/l, pH 9,0) transferiert, mit dem Schraubdeckel verschlossen und 5 sec geschüttelt.

Der Patient sammelt im Mund Speichel und transferiert mindestens 1 ml Speichel in ein 5 ml Plastikröhrchen mit Schraubverschluss. Mit einer Pipette werden 0,6 ml in ein Reaktionsgefäß transferiert in dem 0,25 g Saccharose vorgelegt sind. Mit dem Schraubdeckel wird das Reaktionsgefäß verschlossen und 5 sec geschüttelt.

Nach 3 min wird zur Speichelprobe im Reaktionsgefäß die signalerzeugende Lösung zugegeben. Das verschlossene Reaktionsgefäß wird 5 sec geschüttelt. Nach 3 min wird 0,4 ml Essigsäure-Lösung (Stammlösung Essigsäure 1 mol/l in Wasser) zum Terminieren der Bildung des wahrnehmbaren Signals in das Reaktionsgefäß gegeben. Der Verlauf der Reaktion kann an dem Farbwechsel von gelb nach blau verfolgt werden. Gelb zeigt kein Kariesrisiko an, und je intensiver das Blau ist, desto höher ist das Kariesrisiko.

Bei einer Wellenlänge des eingestrahlten Lichts von 570 nm wurde eine Extinktion ΔE/min von 0,957 mit einem Spektralphotometer UVIKON 930 unter Verwendung einer Kunststoffküvette mit einem Volumen von 1 ml gemessen.

### Vergleichsbeispiel 1a

Vergleichsbeispiel 1a wurde wie Anwendungsbeispiel 1 durchgeführt, es wurde jedoch kein Pyruvat zugegeben. Es wurde eine Extinktion von 3,3 gemessen.

### Vergleichsbeispiel 1b

Vergleichsbeispiel 1 b wurde wie Anwendungsbeispiel 1 durchgeführt, es wurden jedoch weder Pyruvat noch LDH zugegeben. Es wurde eine Extinktion von 0,815 gemessen.

### Vergleichsbeispiel 1c

Vergleichsbeispiel 1 c wurde wie Anwendungsbeispiel 1 durchgeführt, es wurde jedoch keine LDH zugegeben. Es wurde eine Extinktion von 0,156 gemessen.

Die Vergleichsbeispiele 1 a bis 1 c, zusammen mit Anwendungsbeispiel 1, zeigen, daß zwar Pyruvat die LDH inhibiert (vgl. 1 a), aber die Inhibierung durch Pyruvat mittels erhöhter Zugabe an LDH überkompensiert wird (vgl. 1 b) und trotzdem eine oder mehrere unbekannte Nebenreaktionen unterdrückt werden (vgl. 1c).

### Anwendungsbeispiel 2

Zum Anmischen der signalerzeugenden Lösung werden in ein Kunststoffröhrchen (2,5 ml) mit Schraubverschluss mit einer Pipette 0,1 ml NAD-Lösung (Stammlösung 3 mmol/l NAD in Glygly-Puffer 50 mmol/l, pH 9,0), 0,1 ml MTT (Stammlösung 0,15 mmol/l MTT in Glygly-Puffer 50 mmol/l, pH 9,0), 0,1 ml PMS-Lösung (Stammlösung PMS 0,1 mmol/l in Glygly-Puffer 50 mmol/l, pH 9,0), 0,1 ml LDH-Lösung (Stammlösung Lactatdehydrogenase (LDH) 40 Units/ml in Glygly-Puffer 50 mmol/l, pH 9,0), 0,1 ml Pyruvat-Lösung (Stammlösung Pyruvat 2,5 mmol/l Pyruvat in Glygly-Puffer 50 mmol/l, pH 9,0) transferiert, mit dem Schraubdeckel verschlossen und 5 sec geschüttelt.

Mit einer Pipette werden 100 µl auf ein saugfähiges Fließ 2 cm x 0,6 cm x 0,78 mm pipettiert und gefriergetrocknet. Das getrocknete Fließ wird flächig auf ein Ende eines Polyesterstäbchen (7 cm x 0,6 cm x 0,25 mm) geklebt. In einem Reaktionsgefäß wurde eine Speichelprobe, wie unter Anwendungsbeispiel 1 Paragraph 2 beschrieben, vorbereitet. Das Fließ am Teststäbchen wird vollständig in die Speichelprobe eingeführt. Nach 3 min wird das Fließ des Teststreifen in ein Gefäß mit 2 ml Stoppreagenz, bestehend aus einer 50 mmol/l Natriumhydrogenphosphat-Lösung mit einem pH-Wert von 7,5 getaucht. Je höher die Intensität der Blaufärbung des Fließ am Teststäbchen umso höher ist das Kariesrisiko.

### Anwendungsbeispiel 3

Es wird eine signalerzeugende Lösung vorbereitet, wie im Anwendungsbeispiel 2 Paragraph 1 beschrieben. Der Patient lässt einen Würfelzucker im Mund zergehen und sammelt den Speichel im Mund. Ein Wattestäbchen (z.B. Q-Tips) wird in den Mund eingeführt und dort 5 sec belassen, um Speichel aufzusaugen. Der mit Speichel getränkte Wattebereich des Stäbchens wird in die signalerzeugende Lösung eingetaucht und für 3 min dort belassen. Danach wird die entstandene Blaufärbung am Wattebereich des Wattestäbchens evaluiert. Je intensiver die Blaufärbung ist, desto höher ist das Kariesrisiko.

### Anwendungsbeispiel 4

Es wird ein Teststreifen vorbereitet, wie im Anwendungsbeispiel 2 Paragraph 1 beschrieben. Der Patient lässt einen Würfelzucker im Mund zergehen. Der Teststreifen wird in den Mund eingeführt und das saugfähige Vlies mittig auf die Zunge gelegt und gerieben, um Zungenbelag aufzunehmen. Das saugfähige Vlies des Teststreifens wird in die signalerzeugende Lösung eingetaucht und für 3 min dort belassen. Danach wird die entstandene Blaufärbung am Vlies evaluiert. Je intensiver die Blaufärbung, desto höher ist das Kariesrisiko.

### Anwendungsbeispiel 5

In diesem Kit befinden sich die Zutaten, wie in den Anwendungsbeispielen 1 bis 4 beschrieben, um den Speicheltest durchzuführen. Für die diagnostische Abformung befindet sich in einer Tüte 20 g Alginat (Palgat, Fa. ESPE). Das Alginat wird in eine Anmischschale (250 ml) überführt. In einem Gefäß wird eine nach DE-A-199 26 728 angefertigte signalerzeugende Lösung, enthaltend 0,26 g Glycylglycin, 0,24 g Tri-(hydroxymethyl)aminomethan, 36 mg NAD, 0,9 mg Phenazinmethosulfat, 3 mg 3-(4,5-Dimethylthiazolyl-2)-2,5-diphenyltetrazoliumbromid (MTT), 1850 Units Lactatdehydrogenase vorgehalten, mit der die Freisetzung der Milchsäure von kariesauslösenden Mikroorganismen in Plaque beobachtet werden kann. Die signalerzeugende Lösung wird zum Alginat gegeben. Die Mischung wird umgehend mit einem Handspatel bis zur Homogenität gemischt (ca. 30 sec). Während der Bereitstellung der Abformmasse lässt der Patient einen Zuckerwürfel im Mund zergehen. Die Abformmasse wird in einen Abformlöffel eingebracht. Der Abformlöffel wird zur Abdrucknahme entweder an den Ober- oder Unterkiefer angedrückt und dort für 3 min belassen. Die Abformung wird dem Mund entnommen. Die Stellen an den Zähnen, die metabolisch aktive, kariesauslösende Mikroorganismen tragen, werden an blauen Punkten und Flächen an der Abformmasse erkannt. Je intensiver die Blaufärbung, desto höher ist das lokale Kariesrisiko. Das durch den Speicheltest bestimmte Kariesrisiko kann mit dem auf den Zähnen vorhandenen Kariesrisiko verglichen werden.

### Anwendungsbeispiel 6

Ein Blister entsprechend DE 29714246 U kommt zur Anwendung. Es wird ein Wattestäbchen (z.B. Q-Tip) mit einer 50 gew.-%igen Zuckerlösung impregniert. Das Wattestäbchen wird in den Mund eingeführt und mittig auf der Zunge drehend gerieben, um Zungenbelag aufzunehmen. Das Wattestäbchen wird in das erste Reservoir des Blisters eingeführt. Durch Drücken des zweiten Reservoirs wird die signalerzeugende Lösung, hergestellt nach Anwendungsbeispiel 2 Paragraph 1, in das erste Reservoir geleitet. Durch Drehen des Wattestäbchens wird der Teil mit der Speichelprobe mit der signalerzeugenden Lösung benetzt. Das Wattestäbchen kann nach 3 min dem Blister entnommen werden.

### Vergleichsbeispiel 1

Die Vergleichsbeispiele 1 und 2 wurden angefertigt, um zu zeigen, daß die Lehren der Patentschriften US 4,351,899 und US 4,254,222 nicht geeignet sind, das patientenbezogene Kariesrisiko zu bestimmen.

Entsprechend der Lehre der Patentschriften US 4,351,899 und US 4,254,222 wurden zu 100 µl Speichel 0,1 ml Glygly-Pufferlösung pH 9,0 enthaltend 0,6 µmol Lactat, 0,6 µmol NAD, 0,03 µmol MTT, 0,02 µmol PMS und 0,6 Units Lactatdehydrogenase gegeben und durchmischt. Bei 570 nm wurde der Verlauf der Reaktion mit einem Mikroplattenreader (Fa. Molecular Device) verfolgt. Ein Extinktionszunahme von 0,6/min wurde gemessen. In dem Kontrollexperiment ohne Speichel wurde eine Extinktionsänderung von 1,1/min gemessen. Ergebnis: Speichel inhibiert das zur Signalerzeugung benötigte Enzym Lactatdehydrogenase zu 45 %.

### Vergleichsbeispiel 2

Entsprechend der Lehre der Patentschriften US 4,351,899 und US 4,254,222 wurden zu 100 µl Speichel 0,1 ml Glygly-Pufferlösung pH 9,0 enthaltend 0,6 µmol Lactat, 0,6 µmol NAD, 0,03 µmol MTT und 0,02 µmol PMS gegeben und durchmischt. Bei 570 nm wurde der Verlauf der Reaktion mit einem Mikroplattenreader (Fa. Molecular Device) verfolgt. Ohne Gegenwart von Lactatdehydrogenase wurde bereits eine Extinktionszunahme von 0,4/min gemessen. In dem Vergleichsexperiment in Gegenwart von 0,3 Units Lactatdehydrogenase wurde eine Extinktionsänderung von 1,2/min gemessen. Ergebnis: Im Speichel befinden sich Komponenten, die die Redoxindikatoren PMS/MTT bereits reduzieren können und somit das Messsignal zu 50 % verfälschen.

## Patentansprüche

1. Verfahren zur in-vitro Bestimmung des patientenbezogenen Kariesrisikos unter Verwendung von Milchsäure und/oder Lactat in Proben aus dem Mundraum, wobei im Falle von Bioflüssigkeiten zu 0,1 ml Probenvolumen zwischen 0,001 und 100 Units Lactatdehydrogenase zugegeben werden und Pyruvat in Konzentrationen zwischen 0,001 µmol und 5 µmol pro 0,1 ml Probenvolumen zugesetzt wird und im Falle von Biofilmen, die aus 0,01 bis 25 cm² Mundraumoberfläche gewonnen wurden, zwischen 0,001 und 100 Units Lactatdehydrogenase zugegeben werden, und Pyruvat zugesetzt wird und wobei in beiden Fällen NAD in einer Konzentration zwischen 0,01 und 10 mmol/l, bezogen auf die gesamte Testmischung, vorhanden ist und anschließend die entstandene Menge an NADH gemessen wird.

2. Verfahren nach Anspruch 1, bei welchem das Probenvolumen Mikroorganismen enthält, die durch Überführung von Mikroorganismen in den Speichel durch mechanische Einwirkung auf mindestens einen Teil des Mundraums in den Speichel gelangt sind.

3. Verfahren nach Anspruch 2, bei welchem der Teil des Mundraums die Zunge darstellt.

4. Verfahren nach einem der Ansprüche 1 bis 3, bei welchem eine Probe eingesetzt wird, die vor Durchführung der Probennahme mit einer Substanz in Kontakt gebracht wurde, die die Bildung von Milchsäure und/oder Lactat induziert oder vermehrt oder die die Bildung von Milchsäure und/oder Lactat störenden Substanzen behindert oder verhindert.

5. Verfahren nach Anspruch 4, bei welchem vor Durchführung des Nachweisverfahrens mindestens eine Pufferlösung zugesetzt wird.

6. Verfahren nach einem der Ansprüche 1 bis 5, bei welchem nach Entnahme des Probenvolumens aus dem Mundraum der Probe Pyruvat zugesetzt wird.

7. Verfahren zur ortsspezifischen Bestimmung des patientenbezogenen Kariesrisikos, bei welchem ein Verfahren nach einem der vorhergehenden Ansprüche angewandt und anschließend ein Abdruck mittels einer diagnostischen Abformmasse, umfassend ein filmbildendes Trägermaterial und eine Diagnosesubstanz, genommen wird.

8. Kit zur Durchführung des Verfahrens nach einem der Ansprüche 1-7, enthaltend
- mindestens ein Probenröhrchen,
- mindestens eine Aufnahmevorrichtung,
- mindestens eine Puffersubstanz,
- Lactatdehydrogenase in einer Menge, um zwischen 0,001 und 100 Units pro 0,1 ml Probenvolumen zu erzielen,
- NAD in einer Konzentration zwischen 0,01 und 10 mM, bezogen auf die gesamte Testmischung, und
- Pyruvat in einer Menge, um Konzentrationen zwischen 0,001 µmol und 5 µmol pro 0,1 ml Probenvolumen zu erzielen.

## Claims

1. A method for the in-vitro determination of the risk of caries in a patient by using lactic acid and/or lactate in samples from the oral cavity, which method comprises in the case of bioliquids adding between 0.001 and 100 units of lactate dehydrogenase per 0.1 ml of sample volume and adding pyruvate in concentrations between 0.001 µmol and 5 µmol per 0.1 ml of sample volume and in the case of biofilms obtained from 0.01 to 25 cm² of oral cavity surface adding between 0.001 and 100 units of lactate dehydrogenase and adding pyruvate and in both cases NAD being present in a concentration between 0.01 and 10 mmol/l, based on the entire test mixture, and subsequently measuring the resulting amount of NADH.

2. A method according to Claim 1, wherein the sample volume contains micro-organisms which have transferred into the saliva as a result of mechanical action on at least one region of the oral cavity.

3. A method according to Claim 2, wherein the region of the oral cavity is the tongue.

4. A method according to any one of Claims 1 to 3, wherein the sample used is, before the sample is taken, brought into contact with a substance which induces or multiplies the formation of lactic acid and/or lactate or which hinders or prevents the formation of substances that adversely affect lactic acid and/or lactate.

5. A method according to Claim 4, wherein at least one buffer solution is added before the detection method is carried out.

6. A method according to any one of Claims 1 to 5, wherein, after removal of the sample volume from the oral cavity, pyruvate is added to the sample.

7. A method for the site-specific determination of the risk of caries in a patient, which method comprises applying a method according to any one of the preceding claims and subsequently taking an impression using a diagnostic impression compound comprising a film-forming carrier material and a diagnostic substance.

8. A kit for carrying out the method according to any one of Claims 1-7, comprising
- at least one sample tube,
- at least one take-up device,
- at least one buffer substance,
- lactate dehydrogenase in an amount to obtain between 0.001 and 100 units per 0.1 ml of sample volume,
- NAD in a concentration between 0.01 and 10 mM, based on the entire test mixture, and
- pyruvate in an amount to obtain concentrations between 0.001 µmol and 5 µmol per 0.1 ml of sample volume.

## Revendications

1. Procédé destiné à déterminer in vitro le risque de carie chez un patient en utilisant de l'acide lactique et/ou du lactate dans des échantillons prélevés dans la cavité buccale, dans lequel on ajoute par 0,1 ml de volume de l'échantillon, dans le cas de liquides biologiques, entre 0,001 et 100 unités de lactate déshydrogénase et on ajoute du pyruvate dans des concentrations entre 0,001 µmol et 5 µmol par 0,1 ml de volume de l'échantillon alors que, dans le cas de biofilms qui ont été prélevés sur une surface de la cavité buccale comprise entre 0,01 et 25 cm², on ajoute entre 0,001 et 100 unités de lactate déshydrogénase et du pyruvate, du NAD étant présent dans les deux cas, la concentration de ce dernier étant comprise entre 0,01 et 10 mmol/l par rapport à l'intégralité du mélange d'essai, et ensuite la quantité de NADH qui s'est formée étant mesurée.

2. Procédé selon la revendication 1, le volume de l'échantillon contenant des micro-organismes, ces derniers ayant été introduits dans le réservoir en transférant des micro-organismes dans le réservoir par action mécanique sur au moins une partie de la cavité buccale.

3. Procédé selon la revendication 2, ladite partie de la cavité buccale étant la langue.

4. Procédé selon l'une des revendications 1 à 3, comprenant la mise en oeuvre d'un échantillon ayant été mis en contact, au préalable de son prélèvement, avec une substance qui induit ou augmente la formation d'acide lactique et/ou de lactate ou qui inactive ou inhibe les substances empêchant la formation d'acide lactique et/ou de lactate.

5. Procédé selon la revendication 4, au moins une solution tampon étant ajoutée avant la réalisation du procédé de mise en évidence.

6. Procédé selon l'une des revendications 1 à 5, du pyruvate étant ajouté à l'échantillon suite au prélèvement du volume de l'échantillon dans la cavité buccale.

7. Procédé destiné à déterminer, à un endroit spécifique, le risque de carie chez un patient, comprenant la mise en oeuvre d'un procédé selon l'une des revendications précédentes, puis la réalisation d'une empreinte au moyen d'une masse pour la prise d'empreintes, ladite masse comprenant un matériau de support filmogène et une substance aux propriétés diagnostiques.

8. Kit destiné à mettre en oeuvre le procédé selon l'une des revendications 1 à 7, contenant
- au moins un tube d'échantillon,
- au moins un dispositif de réception,
- au moins une substance tampon,
- de la lactate déshydrogénase dans une quantité permettant d'atteindre entre 0,001 et 100 unités par 0,1 ml de volume d'échantillon,
- du NAD dans une concentration comprise entre 0,01 et 10 mM, par rapport à l'intégralité du mélange d'essai, et
- du pyruvate dans une quantité permettant d'atteindre des concentrations comprises entre 0,001 µmol et 5 µmol par 0,1 ml de volume d'échantillon.
